# EUROPEAN PATENT APPLICATION

(11) **EP 3 453 786 A1**
(43) Date of publication of application: **13.03.2019**
(21) Application number: 17190191.1
(22) Date of filing: 08.09.2017
(51) Int. Cl.: C25B 3/02, C25B 3/00, C25B 1/04, C25B 1/30, C25B 9/08, C07C 51/02, C07C 51/285, C07C 59/08

(54) **METHOD FOR PRODUCING LACTIC ACID**

(71) Applicant: Nederlandse Organisatie voor toegepast- natuurwetenschappelijk onderzoek TNO, 2595 DA 's-Gravenhage (NL)
(72) Inventor: Bisselink, Roel Johannes Martinus, 2595 DA 's-Gravenhage (NL); Latsuzbaia, Roman, 2595 DA 's-Gravenhage (NL); Anastasopol, Anca, 2595 DA 's-Gravenhage (NL); Goetheer. Earl Lawrence Vincent, 2595 DA 's-Gravenhage (NL)
(74) Representative: V.O.

(57) **Abstract**

The invention is directed to a method for producing lactate. The method of the invention comprises electrochemically oxidising a catalyst at an anode, and using oxidised catalyst to oxidise propylene glycol and form lactate, thereby reducing the said oxidised catalyst.

## Description

The invention is directed to a method for producing lactate.

Lactic acid (2-hydroxypropionic acid), CH₃CHOHCOOH, is the most widely occurring hydroxycarboxylic acid. Lactic acid is naturally occurring organic acid that can be produced by fermentation or chemical synthesis.

Lactic acid is seen as an important input for the chemical industry, since as well as being used to obtain biodegradable materials it is synthesised from renewable sources, like corn glucose, molasses and cheese whey.

It is present in many foods, both naturally or as a product of *in situ* microbial fermentation (as in sauerkraut, yogurt, buttermilk, sourdough breads, and many other fermented foods). As a food acidulant, lactic acid has a mild acidic taste in contrast to other food acids. Lactic acid is a very good preservative and pickling agent for various food products. Amongst other, it is used as acidulant, flavouring and/or pH buffering agent in a wide variety of processed foods, such as candy, breads and bakery products, soft drinks, soups, sherbets, dairy products, beer, jams and jellies, mayonnaise, and processed eggs, often in conjunction with other acidulants.

Lactic acid is further used in the leather tanning industry as an acidulant for deliming hides and in vegetable tanning. In various textile finishing operations and acid dying of wool, lactic acid is used extensively.

Additional applications of lactic acid include pH adjustment of hardening baths for cellophane that is used in food packaging, terminating agent for phenol-formaldehyde resins, alkyd resin modifier, solder flux, lithographic and textile printing developers, adhesive formulations, electroplating and electropolishing baths, and detergent builders (with maleic anhydride to form carboxymethoxysuccinic acid-type compounds).

Further, lactic acid is used in pharmaceutical and cosmetic applications and formulations, particularly in topical ointments, lotions, parenteral solutions, and biodegradable polymers for medical applications. A substantial part of pharmaceutical lactic acid is used as the sodium salt for parenteral and dialysis applications. The chirality of lactic acid opens further opportunities for the synthesis of drugs and agrochemicals.

Lactic acid can be manufactured by either chemical synthesis or carbohydrate fermentation. Both are used for commercial production. The fermentative production processes require lactic bacteria. These have complex requirements in terms of growth, requiring vitamins and amino acids for their cultivation. Moreover, the fermentative processes are time-consuming, requiring large volumes and creating large quantities of liquid residue which need to be treated.

The chemical synthesis routes produce only the racemic lactic acid. The conventional process is based on lactonitrile, which used to be a by-product from acrylonitrile synthesis. It involves base catalysed addition of hydrogen cyanide to acetaldehyde to produce lactonitrile. The crude lactonitrile is then recovered and purified by distillation and is hydrolysed to lactic acid by using either concentrated hydrochloric of sulphuric acid, producing the corresponding ammonium salt as a by-product. This crude lactic acid is esterified with methanol, producing (i) methyl lactate, which is recovered and purified by distillation and hydrolysed by water under acid catalysts to produce lactic acid, which is further concentrated, and purified, and (ii) methanol, which is recycled.

Other possible chemical routes for lactic acid include, amongst others base catalysed degradation of sugars; reaction of acetaldehyde, carbon monoxide and water at elevate temperatures and pressures; hydrolysis of chloropropionic acid (prepared by chlorination of propionic acid); nitric acid oxidation of propylene; and oxidation of propylene glycol.

Oxidative methods using heterogeneous catalysts have also been disclosed, coming from glycerine as well as 1,2-propanediol.

Some scientific studies deal with the production of pyruvic acid via oxidative routes from 1,2-propanediol, which use platinum or palladium catalysts supported on activated carbon and promoters such as lead, bismuth or tin (Tsujino, J. Mol. Catal. 1992, 71, 25-35 and Pinxt et al., Appl. Catal. A 2000, 191, 45-54). The results of these studies show that lactic acid is produced as a by-product.

In the oxidative processes mentioned above the production of lactic acid takes place from mixtures of glycerine and 1,2-propanediol, using different temperature conditions, total pressure, concentration of the components and the presence of a catalyst or not. Lactic acid and other oxidation products are obtained in these processes.

Many of the oxidative processes of the prior art were developed to promote production of pyruvic acid. Low yield of lactic and the formation of by-products, mainly pyruvic acid and acetic acid, necessarily require the use of additional process steps, which allow the lactic acid to be purified.

EP-A-2 610 238 describes a selective catalytic process with yields of more than 70 % for the production of lactic acid by means of the selective oxidation of 1,2-propanediol, *i.e.,* propylene glycol. The process comprises 1) activation of a noble metal containing catalyst by reducing the noble metal through contact between the catalyst and a gaseous stream containing hydrogen, 2) providing a reactor with the reduced catalyst and an aqueous solution of propylene glycol, 3) bubbling a gaseous stream containing oxygen by means of a mixture of the catalyst and the solution of propylene glycol maintaining the pH at a fixed value and controlling the heating, the stirring and the internal pressure of the reactor, and 4) removing the catalyst by filtration and separating the lactic acid.

Ryabenkova et al., Catal. Today 2013, 203, 139-145 report on the selective oxidation of propylene glycol to lactic acid using mild conditions and gold-platinum nanoparticulate catalysts.

Kochkar et al., J. Catal. 2000, 194, 343-351 disclose the chemical oxidation of propylene glycol to lactic acid using as catalyst 2,2,6,6-tetramethylpiperidine-l-oxyl (TEMPO).

Chadderdon et al., ACS Catal. 2015, 5, 6926-6936 describe selective oxidation of propylene glycol to pyruvate or lactate in electrocatalytic reactors over carbon-supported platinum (Pt/C) and gold (Au/C) anode catalysts.

There remains a need in the art for further methods of producing lactic acid that, for example reduce waste formation of salt and/or use cheaper materials.

Objective of the invention is to address this need and/or to overcome one or more of the disadvantages seen in the prior art.

The inventors found that this objective can be met, at least in part, by synthesising lactate via electrochemical oxidation of propylene glycol.

Accordingly, in a first aspect the invention is directed to a method of producing lactate, the method comprising
- electrochemically oxidising a catalyst at an anode, and
- using oxidised catalyst to oxidise propylene glycol and form lactate, thereby reducing the said oxidised catalyst.

Advantageously, the method of the invention does not require expensive or precious metals, such as gold and/or platinum-group metals. Further, the invention provides for a suitable method of synthesising lactate that only requires a relatively small amounts of chemicals. The produced lactate can simply be converted further to lactic acid.

Furthermore, electrocatalytic oxidation of lactic acid in fuel cell reactors offers the potential economic advantage of coproducing electricity and chemical products simultaneously.

The method of the invention can suitable be carried out in an electrochemical cell comprising an anode in an anode electrolyte solution comprising propylene glycol, wherein the catalyst is immobilised on the anode or wherein the catalyst is dissolved or dispersed in the anode electrolyte, and a cathode in a cathode electrolyte solution, wherein the cathode is in electrical communication with the anode. Catalyst used for the cathodic reaction can be immobilised on the cathode or can be dissolved or dispersed in the cathode electrolyte.

To operate the method, a voltage source is used to apply an anode potential to the anode and a potential difference is created between the anode and the cathode. Driven by this potential difference, electrons flow from the anode to the cathode through an external wire. The electrons at the surface of the cathode undergo reduction reactions with species contained in the cathode electrolyte solutions, while oxidation reactions occur at the anode. In accordance with the invention the catalyst is oxidised at the anode. Oxidised catalyst subsequently reduced by oxidising propylene glycol and being regenerated through oxidation afterwards.

Preferably, the applied anode potential is in the range of 0.4-1.4 V *us.* SCE (saturated calomel electrode), such as 0.7-1.1 V *us.* SCE. The applied anode potential is preferably smaller than the water oxidation potential, *i.e*. -1.23 V *us.* NHE.

The term "catalyst" as used in this application is meant to include heterogeneous catalysts, homogeneous catalysts, as well as mediators. Suitably, the catalyst comprises or is nickel oxide hydroxide (NiOOH), cobalt oxide hydroxide (CoOOH), 2,2,6,6-tetramethylpiperidine-l-oxyl (TEMPO) and/or a derivative thereof, or a bicyclic nitroxyl derivate. Examples of derivatives of 2,2,6,6-tetramethylpiperidine-l-oxyl (TEMPO) include 4-methoxy-2,2,6,6-tetramethylpiperidine-l-oxyl (4-MeO-TEMPO), 4-oxo-2,2,6,6-tetramethylpiperidine-1-oxyl (4-oxo-TEMPO), 4-hydroxy-2,2,6,6-tetramethylpiperidine-1-oxyl (4-hydroxy-TEMPO), 4-benzoyloxy-2,2,6,6-tetra-methylpiperidine-1-oxyl (BnO-TEMPO), 4-acetamido-2,2,6,6-tetramethylpiperidine-1-oxyl (AcNH-TEMPO), 4-acetamino-2,2,6,6-tetramethylpiperidine-1-oxyl (AA-TEMPO), 4-amino-2,2,6,6-tetramethylpiperidine-1-oxyl (4-amino-TEMPO), *N,N*-dimethylamino-2,2,6,6-tetramethylpiperidine-1-oxyl (NNDMA-TEMPO), 3,6-dihydro-2,2,6,6-tetramethyl-1(2H)-pyridinyloxyl (DH-TEMPO), and *bis*(2,2,6,6-tetramethyl-piperidine-1-oxyl-4-yl)sebacate (TINO). Examples of bicyclic nitroxyl derivatives include 2-azaadamantane *N*-oxyl (AZADO) and 9-azabicyclo[3.3.1]nonane *N*-oxyl (ABNO).

Preferably, the catalyst is a homogeneous catalyst. While prior art attempts focussed on heterogeneous catalysts, the present inventors found a method in which a homogeneous catalyst may be employed.

The catalyst may suitably be present in solution. However, it is also possible to use a heterogeneous catalyst, for example one that is immobilised on the electrode. The catalyst can suitably be present on a support, such as an inert solid having high surface area. The support can suitably be a conductive carrier. The solid carrier can, for example, be an inorganic oxide or an organic polymer. Examples of such supports include silica, aluminosilicate, activated carbon, carbon foams, carbon nanotubes, polystyrene, poly(ethyleneimine) silica, celite, carbon, alumina, clays, *etc.* In particular, the TEMPO or derivative thereof, or the bicyclic nitroxyl derivative, can be supported on silica aluminosilicate, polystyrene. WO-A-084800 describes further polymer supports (preferably based on polyolefins, fluorinated polyethylene, cellulose and viscose) containing a functional group capable of reacting with a functionalised TEMPO radical that can be used as solid support.

The catalyst can be essentially free of noble metals. It is further possible that the catalyst is an organic homogeneous catalyst.

The electrolyte solvents in the anode and cathode electrolyte solutions can be the same or different and the electrolyte solutions can be aqueous or non-aqueous. The anode electrolyte and/or the cathode electrolyte can suitably comprise one or more selected from the group consisting of a buffer, sodium perchlorate, sodium sulphate, sodium chloride, sodium perchlorate, sodium bromide, sodium hydroxide, sodium carbonate, sulphuric acid, hydrochloric acid, nitric acid, perchloric acid, potassium sulphate, potassium chloride, potassium perchlorate, potassium bromide, potassium hydroxide, potassium carbonate, ammonium sulphate, ammonium chloride, ammonium perchlorate, ammonium bromide, ammonium hydroxide, ammonium carbonate. Preferably, the electrolyte comprises one or more selected from the group consisting of sodium hydroxide, potassium hydroxide, ammonium hydroxide. It is preferred that the anode electrolyte and/or the cathode electrolyte solutions are essentially free from buffer. Further, it is preferred that the concentration of lactic acid is as high as possible, such as a concentration of 0.1 M or more, or 1.0 M or more. Acidification protonates the formed lactate under formation of lactic acid. The formed sulphuric acid may optionally be reused. In another option, the anode electrolyte and/or the cathode electrolyte solutions comprise ammonium hydroxide. Ammonia may then be recovered as gas, thereby acidifying the solution.

The method of the invention can suitably be carried out at ambient temperatures and pressures. The method can thus suitably be carried out at a temperature of 10 °C or more, such as 15 °C or more, or 20 °C or more. The method of the invention can suitably be carried out at a temperature of 40 °C or less, such as 35 °C or less, or 30 °C or less. The invention may be carried out at a pressure of 51-152 kPa (0.5-1.5 atm), preferably 81-122 kPa (0.8-1.2 atm), such as 91-111 kPa (0.9-1.1 atm).

It is preferred that during the method of the invention, the pH is kept alkaline. For example, the pH may be kept at a value in the range of 9-14, preferably in the range of 9-13, such as in the range of 10-12. In the case of a NiOOH catalyst, the pH value can be 10 or more, whereas in the case of a TEMPO catalyst, or derivative thereof, the pH value can be 9 or more.

The anode used in the method of the invention can comprise one or more materials selected from the group consisting of Au, Ag, carbon, Co, Cr, Cu, Fe, Ir, Mo, Ni, Pb, Pd, Pt, Ru, Ta, Ti, or alloys thereof. Also mixed metal oxides (MMO), that typically consist of Pt, Ir and/or Ru on Ti, with Ta as an intermediate layer can be used as suitable materials for the anode.

The cathode used in the method of the invention can comprise one or more materials selected from the group consisting of Au, carbon, Co, Cr, Cu, Fe, Ir, Mo, Ni, Pb, Pd, Pt, Ru, Ta, Ti or alloys thereof.

The method of the invention can be carried out in a single compartment electrochemical cell comprising at least one anode, at least one cathode and an electrolyte.

It is also possible that the method of the invention is carried out in a two-compartment electrochemical cell, wherein the anode electrolyte solution and the cathode electrolyte solution are separated by a membrane, such as a semi-permeable membrane, a diaphragm or a porous pot. Some examples of commercially available membranes include Nafion™ HP, Nafion™ 211, Nafion™ XL, Nafion™ 212, Nafion™ NE1035, Nafion™ 115, Nafion™ 117, Nafion™ 1110, Nafion™ N324, Nafion™ N424, Nafion™ N438, Fumatech FAS, Fumatech FKS, Fumatech FAB, Fumatech FKB, Fumatech FBM, Fumatech FAD, Fumatech FKD, Fumatech FAP, Fumatech FAA, Fumatech FKL, Fumatech FKE, Neosepta™ CMX, Neosepta™CMS, Neosepta™CMB, Neosepta™AMX, Neosepta™AHA, Neosepta™ACS, Neosepta™AFN, Neosepta™AFX, Neosepta™ACM, Selemion™CMV, Selemion™CMD, Selemion™AMV, Selemion™AMT, Selemion™DSV, Selemion™HSF, Selemion™CSD, Selemion™CMF, Selemion™AAV, Selemion™ASV, Selemion™AHO, and Selemion™APS4.

The oxidised propylene glycol yields lactate, typically sodium lactate, which can be converted into lactic acid in a subsequent step. Such a subsequent step may involve the addition of an acid, preferably a strong acid, such as sulphuric acid. The product is usually a racemic mixture of D,L-lactic acid. However, the method may be adapted to predominantly produce one of the stereoisomers, *i.e.* D-lactic acid or L-lactic acid. For example, D-lactate may be predominantly obtained by using the L-lactate dehydrogenase enzyme (E.C. 1.1.1.27).

In a special embodiment, lactate is formed at the anode by oxidation of propylene glycol. At the cathode, oxygen is reduced to hydrogen peroxide, hydrogen peroxide can subsequently be used for the oxidation of propylene glycol to lactic acid and/or pyruvic acid, so-called paired electrosynthesis.

The method may be performed in a continuous manner. This is particularly advantageous for production on an industrial scale. Such production may, for example, be achieved in an electrochemical cell having an inlet and an outlet. Propylene glycol is fed through the inlet. The pH may be maintained constant by addition of a base, such as sodium hydroxide or potassium hydroxide. Alternatively, or in addition, a buffer may be used to keep the pH constant. The product then comprises sodium lactate which may be collected via the outlet. The sodium lactate can be converted into the desired product lactic acid and sodium hydroxide, which sodium hydroxide can be reused to set the pH in the electrochemical cell.

The invention has been described by reference to various embodiments, compositions and methods. The skilled person understands that features of various embodiments, compositions and methods can be combined with each other. For instance, preferred coating compositions can be used in the various methods, in the same way preferred steps of a method can be combined with each other and with preferred coating compositions.

All references cited herein are hereby completely incorporated by reference to the same extent as if each reference were individually and specifically indicated to be incorporated by reference and were set forth in its entirety herein.

The use of the terms "a" and "an" and "the" and similar referents in the context of describing the invention (especially in the context of the claims) are to be construed to cover both the singular and the plural, unless otherwise indicated herein or clearly contradicted by context. The terms "comprising", "having", "including" and "containing" are to be construed as open-ended terms (*i.e*., meaning "including, but not limited to") unless otherwise noted. Recitation of ranges of values herein are merely intended to serve as a shorthand method of referring individually to each separate value falling within the range, unless otherwise indicated herein, and each separate value is incorporated into the specification as if it were individually recited herein. The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention. For the purpose of the description and of the appended claims, except where otherwise indicated, all numbers expressing amounts, quantities, percentages, and so forth, are to be understood as being modified in all instances by the term "about". Also, all ranges include any combination of the maximum and minimum points disclosed and include any intermediate ranges therein, which may or may not be specifically enumerated herein.

Preferred embodiments of this invention are described herein. Variation of those preferred embodiments may become apparent to those of ordinary skill in the art upon reading the foregoing description. The inventors expect skilled artisans to employ such variations as appropriate, and the inventors intend for the invention to be practiced otherwise than as specifically described herein. Accordingly, this invention includes all modifications and equivalents of the subject-matter recited in the claims appended hereto as permitted by applicable law. Moreover, any combination of the above-described elements in all possible variations thereof is encompassed by the invention unless otherwise indicated herein or otherwise clearly contradicted by context. The claims are to be construed to include alternative embodiments to the extent permitted by the prior art.

For the purpose of clarity and a concise description features are described herein as part of the same or separate embodiments, however, it will be appreciated that the scope of the invention may include embodiments having combinations of all or some of the features described.

### Example

A H-cell, made from glass, was used for electrolysis. An anion exchange membrane was used to separate the compartments. The anolyte (100 ml) consisted of 0.5 M boric acid at pH 9.2 (by addition of NaOH), 7.5 mM TEMPO and 5.0 mM propylene glycol. The catholyte (100 ml) consisted of 0.1 M NaOH. The electrolysis was performed under nitrogen atmosphere and room temperature. The working electrode consisted of a graphite rod, the counter electrode of a spirally wound platinum wire and the reference electrode was a saturated calomel electrode (SCE). Electrolysis was performed at +0.8 V *us.* SCE. Samples were taken during electrolysis and analysed by HPLC running on an Aminex HPX-87H column and a RID detector. The results are shown in the table below.

| Time [hours] | Propylene glycol [mM] | Sodium lactate [mM] |
|---|---|---|
| 0 | 5.26 | 0 |
| 0.35 | 4.03 | 0.53 |
| 0.67 | 3.72 | 0.95 |
| 1 | 3.46 | 1.32 |
| 2 | 2.75 | 2.27 |
| 3 | 2.18 | 2.97 |
| 4 | 1.70 | 3.45 |
| 5 | 1.35 | 3.76 |
| 6 | 1.06 | 4.03 |

After 6 hours of operation 80 % propylene glycol conversion was achieved with a selectivity of 95 % towards sodium lactate.

## Claims

1. Method of producing lactate, the method comprising
- electrochemically oxidising a catalyst at an anode, and
- using oxidised catalyst to oxidise propylene glycol and form lactate, thereby reducing the said oxidised catalyst.

2. Method according to claim 1 wherein the catalyst is chosen from the group consisting of NiOOH, CoOOH, bicyclic nitroxyl derivates, 2,2,6,6-tetramethylpiperidine-1-oxyl (TEMPO) and/or a derivative thereof.

3. Method according to claim 1 or 2, wherein the catalyst is an organic homogeneous catalyst.

4. Method according to any one of claims 1-3, which method is carried out at a temperature of 10-40 °C, such as 15-35 °C.

5. Method according to any one of claims 1-4, wherein the pH is kept within the range of 9-14, preferably in the range of 10-12.

6. Method according to any one of claim 1-5, wherein the cathode comprises a material selected from the group consisting of Au, carbon, Co, Cr, Cu, Fe, Ir, Mo, Ni, Pb, Pd, Pt, Ru, Ta, Ti and alloys thereof.

7. Method according to any one of claims 1-6, wherein the anode comprises a material selected from the group consisting of Au, Ag, carbon, Co, Cr, Cu, Fe, Ir, Mo, Ni, Pb, Pd, Pt, Ru, Ta, Ti, and alloys thereof.

8. Method according to any one of claims 1-7, wherein an anode potential of 0.4-1.4 V *us.* SCE is applied, such as 0.7-1.1 V *us.* SCE.

9. Method according to any one of claims 1-8, wherein said method is carried out in an electrochemical cell comprising
- the anode in an anode electrolyte solution comprising propylene glycol, wherein the catalyst is immobilised on the anode or is dissolved or dispersed in the anode electrolyte, and
- a cathode in a cathode electrolyte solution, wherein the cathode is in electrical communication with the anode.

10. Method according to any one of claim 1-9, which method is carried out in a two-compartment electrochemical cell wherein the anode electrolyte solution and the cathode electrolyte solution are separated by a membrane, such as a semi-permeable membrane, a diaphragm or a porous pot.

11. Method according to any one of claims 1-10, wherein said method further comprises a step of converting produced lactate into lactic acid, such as by adding an acid.

12. Method according to claim 11, wherein the method is adapted to predominantly produce D-lactic acid or L-lactic acid, such as by using L-lactate dehydrogenase enzyme (E.C. 1.1.1.27).

13. Method according to any one of claims 1-12, wherein molecular hydrogen is formed at the cathode by reduction of water.

14. Method according to any one of claims 1-13, wherein lactate is formed at the anode by oxidation of propylene glycol and oxygen is reduced to hydrogen peroxide at the cathode.

15. Method according to claim 14, wherein said hydrogen peroxide is used for the oxidation of propylene glycol to lactic acid and/or pyruvic acid.
